(19) Europäisches Patentamt
European Patent Office
Office européen des brevets

(11) **EP 2 201 098 B1**

(12) **EUROPEAN PATENT SPECIFICATION**

(45) Date of publication and mention
of the grant of the patent:
**28.08.2013 Bulletin 2013/35**

(51) Int Cl.:
*C12N 1/34* (2006.01)  *C12N 1/00* (2006.01)
*C12P 1/00* (2006.01)

(21) Application number: **08840309.2**

(86) International application number:
**PCT/EP2008/063930**

(22) Date of filing: **16.10.2008**

(87) International publication number:
**WO 2009/050222 (23.04.2009 Gazette 2009/17)**

(54) **METHOD FOR PRODUCING A FOAMING AGENT**

VERFAHREN ZUR HERSTELLUNG EINES SCHAUMBILDNERS

PROCÉDÉ DE PRODUCTION D'UN AGENT MOUSSANT

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **18.10.2007 EP 07118762**

(43) Date of publication of application:
**30.06.2010 Bulletin 2010/26**

(73) Proprietors:
• **Unilever PLC**
**London EC4Y 0DY (GB)**
Designated Contracting States:
**CY GB IE MT**
• **Unilever N.V.**
**3013 AL Rotterdam (NL)**
Designated Contracting States:
**AT BE BG CH CZ DE DK EE ES FI FR GR HR HU IS IT LI LT LU LV MC NL NO PL PT RO SE SI SK TR**

(72) Inventors:
• **COX, Andrew, Richard**
**Bedford, Bedfordshire MK44 1LQ (GB)**
• **RUSSELL, Andrew, Baxter**
**Bedford, Bedfordshire MK44 1LQ (GB)**
• **TIER, Christopher, Mark**
**Bedford, Bedfordshire MK44 1LQ (GB)**

(74) Representative: **Hugot, Alain**
**Unilever PLC, Unilever Patent Group**
**Colworth House**
**Sharnbrook, Bedford**
**Bedfordshire MK44 1LQ (GB)**

(56) References cited:
**EP-A- 0 216 270    US-A- 4 946 625**

**US-A- 4 960 540    US-B1- 6 238 714**

• CHAISALEE R ET AL: "MECHANISM OF ANTIFOAM BEHAVIOR OF SOLUTIONS OF NONIONIC SURFACTANTS ABOVE THE CLOUD POINT" JOURNAL OF SURFACTANTS AND DETERGENTS, AOCS PRESS, CHAMPAIGN, IL, US, vol. 6, no. 4, October 2003 (2003-10), pages 345-351, XP001176159 ISSN: 0024-4201
• MCGREGOR M C ET AL: "ANTIFOAM EFFECTS ON ULTRAFILTRATION" BIOTECHNOLOGY AND BIOENGINEERING, vol. 31, no. 4, 1988, pages 385-389, XP002463343 ISSN: 0006-3592
• COLLEN ANNA ET AL: "A novel two-step extraction method with detergent/polymer systems for primary recovery of the fusion protein endoglucanase I-hydrophobin I" BIOCHIMICA ET BIOPHYSICA ACTA, vol. 1569, no. 1-3, 15 January 2002 (2002-01-15), pages 139-150, XP004341311 ISSN: 0006-3002
• HUNG,K-C. ET AL.: "Cloud-point extraction of selected polycyclic aromatic hydrocarbons by nonionic surfactants" SEPARATION PURIFICATION TECHNOLOGY, vol. 57, 20 August 2007 (2007-08-20), pages 1-10, XP022207795
• HOLMES W., SMITH R., BILL R.: "Evaluation of antifoams in the expression of a recombinant FC fusion protein in shake flask cultures of Saccharomyces cerevisiae & Pichia pastoris" MICROBIAL CELL FACTORIES, vol. 5, no. 1, 2006, page P30, XP002512428

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 201 098 B1

• BAILEY M J ET AL: "Process Technological effects of deletion and amplification of hydrophobins I and II in transformants of Trichoderma reesei" APPLIED MICROBIOLOGY AND BIOTECHNOLOGY, SPRINGER VERLAG, BERLIN, DE, vol. 58, 1 January 2002 (2002-01-01), pages 721-727, XP003015238 ISSN: 0175-7598 cited in the application

• LINDER MARKUS ET AL: "The hydrophobins HFBI and HFBII from Trichoderma reesei showing efficient interactions with nonionic surfactants in aqueous two-phase systems" BIOMACROMOLECULES, vol. 2, no. 2, July 2001 (2001-07), pages 511-517, XP002512510 ISSN: 1525-7797

**Description**

**Technical Field of the Invention**

The present invention relates to industrial fermentation methods. In particular it relates to the extra-cellular production of a foaming agent by fermentation.

**Background to the invention**

Foaming is a common problem in aerobic, submerged fermentations. Foaming is caused by the sparging of gas into the fermentation medium for the purpose of providing oxygen for the growth of the aerobic organism being cultivated (e.g. bacteria, yeasts, fungi, algae, cell cultures). If the fermentation medium contains surface active components such as proteins, polysaccharides or fatty acids, then foam can be formed on the surface of the medium as the sparged gas bubbles disengage from the liquid. Foaming creates a number of problems including the undesirable stripping of product, nutrients, and cells into the foam, and can make process containment difficult. A known method for controlling foaming is to use antifoams, of which several types are commonly used: silicone-based (e.g. polydimethylsiloxanes), polyalkylene glycols (e.g. polypropylene glycol), fatty acids, polyesters and natural oils (e.g. linseed oil, soybean oil). Antifoams replace foam-forming components on bubble surfaces, resulting in destruction of the foam by bubble coalescence. Antifoams are added at the start of and / or during the fermentation.

When the fermentation product is intended for use in foods, personal products or medicine, it is highly desirable that the product is excreted by the producing organism into the fermentation medium (i.e. extra-cellular, rather than intra-cellular production). This avoids the need to disrupt the cells by physical or chemical means in order to release the product for recovery. By maintaining the cells intact, the cellular material can be easily separated from the product so that it is free of intracellular and genetic material which is usually regarded as an undesirable contaminant. This can be especially important when the producing organism has been genetically modified. However, extra-cellular production may intensify the degree of foaming in the fermenter, especially if the product facilitates foam formation or enhances foam stability, for example a biosurfactant or a hydrophobin. The use of antifoams presents a particular problem in the extra-cellular production of such foaming agents for two reasons: firstly the amount of antifoam required is increased because the foaming agent itself contributes to foaming in the fermenter. Secondly, it is not necessary to remove the antifoam from most fermentation products since it is present in low concentrations which do not affect the functionality of the product. However, when the fermentation product is a foaming agent, the antifoam must be substantially removed since the presence of antifoam in the product will impair its functionality.

Bailey et al, Appl. Microbiol. Biotechnol. 58 (2002) pp 721-727 disclose the production of hydrophobins HFB I and HFB II by the fermentation of transformants of *Trichoderma reesei.* An antifoam (Struktol J633) was used to prevent foaming and the hydrophobin was purified using aqueous two phase extraction. However separation methods such as aqueous two phase extraction or chromatographic processes are expensive and may require food-incompatible chemicals.

Davis et al, Enzyme and Microbial Technology 28 (2001) pp 346-354 disclose an alternative method which avoids the need for antifoams. In this method the foam produced during fermentation is collected, and the product recovered from it. This method was successfully applied for the recovery and concentration of surfactin, a lipopeptide biosurfactant. However, this method has a number of drawbacks: firstly, continuous removal of the foam could compromise the aseptic nature of the fermentation; secondly, removal of the foam could affect the viable cell count (because some cells could be carried over with the foam), the liquid volume and nutrient level in the fermenter, making control of the fermentation more difficult; and thirdly, extraction of the product from the foam could be difficult, especially when the product forms very stable foams. Thus there remains a need for an improved fermentation method for extra-cellular production of foaming agents.

**Brief Description of the Invention**

We have now found that by using a specific group of antifoams to suppress foaming in the extra-cellular production of foaming agents by fermentation, the antifoam can be easily removed from the product. Accordingly, in a first aspect, the present invention provides a method for producing a foaming agent comprising:

    i) cultivating a host cell in a fermentation medium wherein:

        the host cell extra-cellularly secretes a foaming agent; and
        the fermentation medium contains an antifoam which has a cloud point;

ii) removing the antifoam while the temperature of the fermentation medium is above the cloud point.

Using an antifoam minimises foaming during fermentation. Selecting an antifoam which has a cloud point and ensuring that the temperature of the fermentation medium is above this cloud point causes the antifoam to "cloud out" (precipitate) in particulate form. This provides a simple route by which the antifoam can be removed after the fermentation has completed, for example by filtration, centrifugation or adsorption. In contrast, antifoams which do not have a cloud point require more complex and / or expensive separation processes such as aqueous two phase extraction or chromatography.

Preferably in step i) the fermentation medium is aerated by sparging air or oxygen-enriched air into it.

Preferably in step i) the temperature of the fermentation medium is above the cloud point of the antifoam.

Preferably in step ii) the antifoam is removed by filtration, centrifugation or adsorption. More preferably; the antifoam is removed by membrane (cross-flow) filtration.

Preferably in step ii) at least 75% of the antifoam is removed, more preferably at least 85%, most preferably at least 90%.

Preferably in step ii), the temperature of the fermentation medium is at least 10°C above the cloud point, more preferably at least 20°C above the cloud point, most preferably at least 30°C above the cloud point.

Preferably the host cells are also removed from the fermentation medium in step ii).

Preferably the foaming agent is purified and/or concentrated from the fermentation medium after step ii), for example by ultrafiltration.

Preferably the antifoam is food-grade.

Preferably, the antifoam comprises at least one non-ionic surfactant/polymer, such as a polyether, a poly (alkylene glycol), an ethylene/propylene oxide block co-polymer, a polyalcohol based on an ethylene/propylene oxide block co-polymer, a polypropylene glycol-based polyether dispersion, or an alkoxylated fatty acid ester.

Preferably the foaming agent is food grade.

Preferably the foaming agent is a hydrophobin, more preferably a class II hydrophobin, most preferably HFBI or HFBII from *Trichoderma reesei.*

Preferably the host cell is a genetically-modified fungus, more preferably a yeast, most preferably *Saccharomyces cerevisiae.*

Preferably, after step ii), the weight ratio of antifoam to foaming agent is less than 0.2, more preferably less than 0.15, most preferably less than 0.1.

**Detailed Description of the Invention**

Unless defined otherwise, all technical and scientific terms used herein have the same meaning as commonly understood by one of ordinary skill in the art (e.g. in cell culture, molecular genetics, nucleic acid chemistry, hybridisation techniques and biochemistry). Standard techniques used for molecular and biochemical methods can be found in Sambrook et al., Molecular Cloning: A Laboratory Manual, 3rd ed. (2001) Cold Spring Harbor Laboratory Press, Cold Spring Harbor, N.Y. and Ausubel et al., Short Protocols in Molecular Biology (1999) 4th Ed, John Wiley & Sons, Inc. - and the full version entitled Current Protocols in Molecular Biology.

Foaming agents

In the context of the present invention, the term "foaming agent" means a surfactant of biological origin which facilitates foam formation and / or enhances its stability by inhibiting the coalescence of bubbles.

Preferably the foaming agent is such that in aqueous solution, the foaming agent produces a foam having a gas phase volume of at least 20% of which at least 50% remains after storage for 1 at 5°C, more preferably after 2 hours, most preferably after 4 hours, according to the following test.

80 mL of an aqueous solution of foaming agent (0.5 wt. %) is prepared. The solution is aerated by shearing the solution in a cooled (2°C) cylindrical, vertically mounted, jacketed stainless steel vessel with internal proportions of 105 mm height and diameter 72 mm. The lid of the vessel fills 54% of the internal volume leaving 46% (180 ml) for the sample. The rotor used to shear the sample consists of a rectangular impeller of the correct proportions to scrape the inside surface of the container as it rotates (72 mm x 41.5mm). Also attached to the rotor are two semi-circular (60 mm diameter) high-shear blades positioned at a 45° angle to the rectangular attachment. 80mL solution is poured into the vessel and the lid secured. The solution is then sheared at 1250 rpm for 10 minutes. The aerated solution is immediately poured into a measuring cylinder. The foam volume is read off from the measuring cylinder immediately, and again after storage at 5°C. The gas phase volume is determined from the measured foam volume and the known volume of the aqueous phase (i.e. 80 mL) as follows:

$$\text{gas phase volume} = [(\text{foam volume} - 80\text{mL}) / \text{foam volume})] \times 100$$

The liquid in the foam drains over time, leading to two separate and distinct layers: a foam on top, and aqueous solution below. However, it is the stability of the foam phase that is the point of interest here. For the calculation of gas phase volume, the volume of foam is taken as the entire volume of the system, i.e. both gas phase and liquid phase irrespective of whether they have separated into two distinct layers. The value of gas phase volume therefore gives a quantitative indication of the stability of the foam to loss of gas. Thus if the initial gas phase volume of the foam is 50%, then after storage the gas phase volume should be at least 25%; if the initial gas phase volume is 20%, then after storage it must be at least 10%.

Foaming agents include hydrophobins and biosurfactants such as glycolipids (e.g. rhamnolipids, trehalolipids cellobiolipids, sophorolipids); lipopeptides and lipoproteins (e.g. peptide-lipid, serrawettin, viscosin, surfactin, subtilisin, gramicidins, polymyxins); fatty acids, neutral lipids, and phospholipids; polymeric biosurfactants (e.g. emulsan, biodispersan, mannan-lipid-protein, liposan, carbohydrate-protein-lipid, protein PA), particulate biosurfactants (vesicles and fimbriae, whole cells), glycosides (e.g. saponins) and fibrous proteins (e.g. fibroin). Dairy and soy proteins / protein hydrolysates are also foaming agents, although these are not usually produced by fermentation methods. Preferably the foaming agent is not a dairy or soy protein or protein hydrolysate. In a particularly preferred embodiment, the foaming agent is a hydrophobin.

Foaming agents can be obtained by culturing host organisms that naturally secrete the foaming agent into the fermentation medium. For example, hydrophobins can be obtained by culturing filamentous fungi such as hyphomycetes (e.g. *Trichoderma)*, basidiomycetes and ascomycetes. Particularly preferred hosts are food grade organisms, such as *Cryphonectria parasitica* which secretes a hydrophobin termed cryparin (MacCabe and Van Alfen, 1999, App. Environ. Microbiol 65: 5431-5435). Similarly, surfactin can be obtained from *Bacillus subtilis* and glycolipids from e.g. *Pseudomanas aeruginosa, Rhodococcus erythropolis, Mycobacterium* species and *Torulopsis bombicola* (Desai and Banat, Microbiology and Molecular Biology Reviews, Mar. 1997, pp 47-64).

Alternatively, foaming agents can be produced by the use of recombinant technology. For example host cells, typically micro-organisms, may be modified to express foaming agents. Techniques for introducing nucleic acid constructs encoding foaming agents (where the foaming agent is a polypeptide) or enzymes necessary to produce foaming agents (where the foaming agent is non-peptide e.g. a biosurfcatant) into host cells are well known in the art. Recombinant technology can also be used to modify foaming agent sequences or synthesise novel foaming agents having desired/ improved properties.

Typically, an appropriate host cell or organism is transformed by a nucleic acid construct that encodes for a desired polypeptide foaming agent. The nucleotide sequence coding for the polypeptide can be inserted into a suitable expression vector encoding the necessary elements for transcription and translation and in such a manner that they will be expressed under appropriate conditions (e.g. in proper orientation and correct reading frame and with appropriate targeting and expression sequences). The methods required to construct these expression vectors are well known to those skilled in the art.

A number of expression systems may be used to express the polypeptide coding sequence. These include, but are not limited to, bacteria, fungi (including yeast), insect cell systems, and plant cell culture systems that have been transformed with the appropriate expression vectors. Preferred hosts are those that are considered food grade -'generally regarded as safe' (GRAS).

Suitable fungal species, include yeasts such as (but not limited to) those of the genera *Saccharomyces, Kluyveromyces, Pichia, Hansenula, Candida, Schizosaccharomyces* and the like, and filamentous species such as (but not limited to) those of the genera *Aspergillus, Trichoderma, Mucor, Neurospora, Fusarium* and the like.

The sequences encoding polypeptide foaming agents are preferably at least 80% identical at the amino acid level to a foaming agent identified in nature, more preferably at least 95% or 100% identical. However, persons skilled in the art may make conservative substitutions or other amino acid changes that do not reduce the biological activity of the foaming agent.

Hydrophobins are a particularly preferred class of foaming agent. In EP 1 623 631 we have previously found that hydrophobins allow the production of aqueous foams with excellent stability to disproportionation and coalescence. Because hydrophobins are highly effective foaming agents, their presence in the fermentation medium presents a particular challenge for foam control.

Hydrophobins are a well-defined class of proteins (Wessels, 1997, Adv. Microb. Physio. 38: 1-45; Wosten, 2001, Annu Rev. Microbiol. 55: 625-646) capable of self-assembly at a hydrophobic/hydrophilic interface, and having a conserved sequence:

$$X_n\text{-}C\text{-}X_{5\text{-}9}\text{-}C\text{-}C\text{-}X_{11\text{-}39}\text{-}C\text{-}X_{8\text{-}23}\text{-}C\text{-}X_{5\text{-}9}\text{-}C\text{-}C\text{-}X_{6\text{-}18}\text{-}C\text{-}X_m \text{ (SEQ ID No. 1)}$$

where X represents any amino acid, and n and m independently represent an integer. Typically, a hydrophobin has a length of up to 125 amino acids. The cysteine residues (C) in the conserved sequence are part of disulphide bridges. In the context of the present invention, the term hydrophobin has a wider meaning to include functionally equivalent proteins still displaying the characteristic of self-assembly at a hydrophobic-hydrophilic interface resulting in a protein film, such as proteins comprising the sequence:

$$Xn\text{-}C\text{-}X_{1\text{-}50}\text{-}C\text{-}X_{0\text{-}5}\text{-}C\text{-}X_{1\text{-}100}\text{-}C\text{-}X_{1\text{-}100}\text{-}C\text{-}X_{1\text{-}50}\text{-}C\text{-}X_{0\text{-}5}\text{-}C\text{-}X_{1\text{-}50}\text{-}C\text{-}X_m \text{ (SEQ ID No. 2)}$$

or parts thereof still displaying the characteristic of self-assembly at a hydrophobic-hydrophilic interface resulting in a protein film. In accordance with the definition of the present invention, self-assembly can be detected by adsorbing the protein to Teflon and using Circular Dichroism to establish the presence of a secondary structure (in general, $\alpha$-helix) (De Vocht et al., 1998, Biophys. J. 74: 2059-68).

The formation of a film can be established by incubating a Teflon sheet in the protein solution followed by at least three washes with water or buffer (Wosten et al., 1994, Embo. J. 13: 5848-54). The protein film can be visualised by any suitable method, such as labeling with a fluorescent marker or by the use of fluorescent antibodies, as is well established in the art. m and n typically have values ranging from 0 to 2000, but more usually m and n in total are less than 100 or 200. The definition of hydrophobin in the context of the present invention includes fusion proteins of a hydrophobin and another polypeptide as well as conjugates of hydrophobin and other molecules such as polysaccharides.

Hydrophobins identified to date are generally classed as either class I or class II. Both types have been identified in fungi as secreted proteins that self-assemble at hydrophobilic interfaces into amphipathic films. Assemblages of class I hydrophobins are generally relatively insoluble whereas those of class II hydrophobins readily dissolve in a variety of solvents. Preferably the hydrophobin is soluble in water, by which is meant that it is at least 0.1% soluble in water, preferably at least 0.5%. By at least 0.1% soluble is meant that no hydrophobin precipitates when 0.1g of hydrophobin in 99.9 mL of water is subjected to 30,000 g centrifugation for 30 minutes at 20°C.

Hydrophobin-like proteins (e.g."chaplins") have also been identified in filamentous bacteria, such as *Actinomycete* and *Streptomyces* sp. (WO01/74864; Talbot, 2003, Curr. Biol, 13: R696-R698). These bacterial proteins by contrast to fungal hydrophobins, may form only up to one disulphide bridge since they may have only two cysteine residues. Such proteins are an example of functional equivalents to hydrophobins having the consensus sequences shown in SEQ ID Nos. 1 and 2, and are within the scope of the present invention.

More than 34 genes coding for hydrophobins have been cloned, from over 16 fungal species (see for example WO96/41882 which gives the sequence of hydrophobins identified in *Agaricus bisporus;* and Wosten, 2001, Annu Rev. Microbiol. 55: 625-646). For the purpose of the invention hydrophobins possessing at least 80% identity at the amino acid level to a hydrophobin that naturally occurs are also embraced within the term "hydrophobins".

Antifoams

The term "antifoam" includes both antifoams which are usually added before foaming occurs and also those which are usually added once the foam has formed (sometimes known as defoamers). The specific group of antifoams suitable for use in the present invention are those that exhibit a cloud point. The cloud point is the temperature at which an aqueous solution of the antifoam becomes visibly turbid as it phase separates (i.e. the antifoam molecules form aggregates which scatter light) as described on p63 of Surfactant Aggregation and Adsorption at Interfaces, J. Eastoe, in Colloid Science: Principles, Methods and Applications, ed. T. Cosgrove, Blackwell Publishing, 2005.

Examples of antifoams which display cloud points include poly(alkylene glycol) (PAG) based compounds such as ethylene oxide/propylene oxide block copolymers, polyalcohols based on ethylene oxide/propylene oxide block co-polymers and polyethers of ethylene and propylene oxides; and fatty acid ester-based compounds.

The cloud point depends on the surfactant composition and chemical structure. For example, for polyoxyethylene (PEO) non-ionic surfactants, the cloud point increases as the EO content increases for a given hydrophobic group. Preferably the cloud point of the antifoam is between 0°C and 90°C, more preferably between 5°C and 60°C.

Preferably, the antifoam comprises at least one non-ionic surfactant/polymer, such as a polyether, a poly (alkylene glycol), an ethylene/propylene oxide block co-polymer, a polyalcohol based on an ethylene/propylene oxide block co-polymer, a polypropylene glycol-based polyether dispersion, or an alkoxylated fatty acid ester. PAG-based antifoams (such as Struktol J647 obtainable from Schill and Seilacher), polyalcohols based on EO/PO block co-polymers (such as Struktol J647 obtainable from Schill and Seilacher) and other non-ionic surfactant antifoams are particularly effective at destroying foam, even in the presence of powerful foaming agents such as hydrophobin.

Mixtures of antifoams can be used, in which case, the cloud point of such a mixture is defined as the highest cloud point of the individual components.

Some common commercially available antifoams that exhibit a cloud point are shown in Table 1.

Table 1

| Antifoam | Cloud Point / °C |
|---|---|
| **Poly(alkylene glycol)** | |
| Struktol J647, Schill & Seilacher | 24 |
| Struktol SB2121 | *ca.* 30 |
| UCON LB 65, Dow Chemical Company | 25 |
| UCON LB 285 | 15 |
| UCON LB 625 | 10 |
| UCON LB 1715 | 8 |
| KFO673, Lubrizol | 25 |
| ST934, Pennwhite Ltd | *ca.* 20 |
| **Ethylene/propylene oxide block co polymers** | |
| Pluronic PE3100, BASF | 41 |
| Pluronic PE6100 | 23 |
| Pluronic PE6200 | 33 |
| Pluronic PE8100 | 36 |
| Pluronic PE10100 | 35 |
| Mazu DF204, BASF | 18-21 |
| **Polyalcohol based on EO/PO block co polymer** | |
| Struktol J650, Schill & Seilacher | 13 |
| **Polypropylene glycol based polyether dispersions** | |
| Antifoam 204, Sigma | 15 |
| **Alkoxylated fatty acid ester** | |
| Struktol J673, Schill & Seilacher | 30 |

Fermentation process and removal of the anitfoam

The fermentation to produce the foaming agent is carried out by culturing the host cell in a liquid fermentation medium within a bioreactor (e.g. an industrial fermenter). The composition of the medium (e.g. nutrients, carbon source etc.), temperature and pH are chosen to provide appropriate conditions for growth of the culture and/or production of the foaming agent. Air or oxygen-enriched air is normally sparged into the medium to provide oxygen for respiration of the culture.

The antifoam may be included in the initial medium composition and/or added as required through the period of the fermentation. Common practice is to employ a foam detection method, such as a conductivity probe, which automatically triggers addition of the antifoam. In the present invention, the antifoam is preferably present at a concentration of from 0.1 to 20g/L, more preferably from 1 to 10g/L. The fermenter temperature during step i), i.e. during fermentation, may be above or below the cloud point of the antifoam. Preferably the fermenter temperature is above the cloud point of the antifoam, since the antifoam is most effective at causing bubble coalescence and foam collapse above its cloud point. The fermenter temperature is generally chosen to achieve optimum conditions for growth of the host cells and / or production.

At the end of the fermentation, the antifoam must be substantially removed to ensure that the functionality of the foaming agent is not impaired. Preferably at least 75% of the antifoam is removed, more preferably at least 85%, most preferably at least 90%. For example, after step ii) the weight ratio of antifoam to foaming agent is preferably less than 0.2, more preferably less than 0.15, most preferably less than 0.1.

Removal of the antifoam is achieved by ensuring that the temperature of the fermentation medium is above the cloud point of the antifoam, so that the antifoam phase separates. The phase separated antifoam can be removed from the fermentation medium by any suitable method such as:

- filtration, e.g. dead-end filtration or a filter press
- membrane (cross-flow) filtration, e.g. microfiltration or ultrafiltration
- centrifugation
- adsorption, using e.g. activated carbon, silica or diatomaceous earth as an absorbent.

The removal of the antifoam may take place by e.g. one of these processes in a single step. Alternatively, the processes may be repeated or combined. For example, after a first filtration step, the filtrate may be re-heated (if necessary) and filtered again.

We have found that more antifoam is removed if the temperature of the fermentation medium is at least 10°C above the cloud point, preferably at least 20°C above the cloud point, most preferably at least 30°C above the cloud point. The temperature of the fermentation medium must not be so high that the foaming agent is denatured. For this reason, it is preferable that the foaming agent is heatstable, e.g. hydrophobins. Preferably the temperature of the fermentation medium is less than 90°C, more preferably less than 75°C. In a preferred embodiment, the antifoam has a cloud point in the range 20-30°C and the temperature of the fermentation medium in step ii) is in the range 40-60°C. By contrast, in conventional processes holding the fermentation medium at such an elevated temperature is deliberately avoided, in order to minimise the possibility of degradation reactions (which can cause colour and flavour changes), enzyme inactivation, protein denaturation and loss of, functionality (see for example, page 7 of "Separation Processes in the Food and Biotechnology Industries", Eds. Grandison, A.S.; Lewis, M.J.).

A preferred method for separating the antifoam is membrane filtration. It has been generally thought that carrying out membrane filtration of fermentation broths containing an antifoam at temperatures above its cloud point results in fouling of the membrane by the precipitated antifoam, causing a low permeate flux and consequent processing difficulties: see for example Yamagiwa et al., J. Chem. Eng. Japan, 26 (1993) pp 13-18, and WO 01 / 014521. Thus it has previously been thought that membrane filtration should take place at temperatures below the cloud point. However, we have now found that acceptable fluxes are obtained when carrying out ultrafiltration and microfiltration operations at a temperature of about 25°C above the cloud point of the antifoam.

In order to ensure that the product foaming agent is free from of intracellular and genetic material (which is usually regarded as an undesirable contaminant) the cells must be removed from the fermentation medium. In a preferred embodiment, the cells are separated from the medium at the same time as the precipitated antifoam is removed, for example in a microfiltration step which takes place at a temperature above the cloud point.

In an alternative embodiment the cells may be removed from the medium in a separate step prior to the removal of the antifoam - for example by filtration (e.g. dead-end filtration or a filter press), membrane / cross-flow filtration, (e.g. microfiltration or ultrafiltration), or centrifugation - at a temperature below the cloud point. In this embodiment, a purification and/or concentration step (e.g. by ultrafiltration) may be carried out (again at a temperature below the cloud point) after cell removal but before antifoam separation. The medium is then heated to a temperature above the cloud point so that the antifoam can be removed as already described.

Once the antifoam and the cells have been removed from the fermentation medium, the product foaming agent may be further purified and concentrated as required, e.g. by ultrafiltration. If the foaming agent is a hydrophopbin, it can be purified from the fermentation medium by, for example, the procedure described in WO01/57076 which involves adsorbing the hydrophobin to a surface and then contacting the surface with a surfactant, such as Tween 20, to elute the hydrophobin from the surface. See also Collen et al., 2002, Biochim Biophys Acta. 1569: 139-50; Calonje et al., 2002, Can. J. Microbiol. 48: 1030-4; Askolin et al., 2001, Appl Microbiol Biotechnol. 57: 124-30; and De Vries et al., 1999, Eur J Biochem. 262: 377-85.

The present invention will now be further described with reference to the following examples which are illustrative only and non-limiting, and the figures wherein:

Figure 1 shows the % transmission as a function of temperature for 0.2 wt% aqueous solutions of Struktol J647 and J633.

Figure 2 shows the calibration graph determined in example 2.

**Examples**

Example 1: Cloud point determination for antifoams

The cloud point of an antifoams is measured by the following method, demonstrated here for two commercially available antifoams, one of which has a cloud point (Struktol J647) and one which does not (Struktol J633).

A solution of 0.2 wt% of each antifoam was prepared in aqueous solution at room temperature. 20mL samples were poured into a cylindrical glass vials (Turbiscan). The samples were equilibrated at the measurement temperature in the water bath for 1 hour. The turbidity of the sample was determined using a Turbiscan Lab Expert (Formulaction, France). This instrument has a light source with a wavelength $\lambda$ of 880nm and an optical sensor 180° from the incident light which measures the percentage of the incident light that is transmitted through the sample at a point 25mm from the base of the vial containing the sample solution. As the solution becomes more turbid, the transmitted light reduces. Sample vials were transferred to the Turbiscan Lab Expert which was also set at the desired measurement temperature.

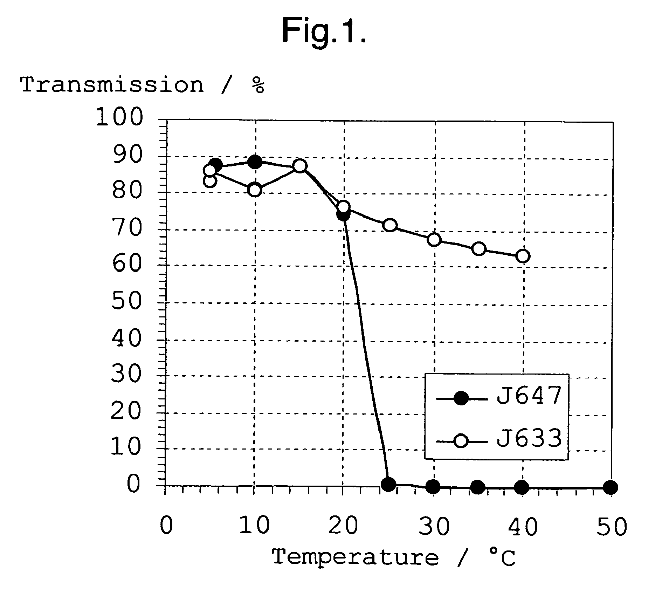

The % transmission was measured as a function of temperature at 5°C intervals, starting from 5°C, and the results are shown in Figure 1. For J647, the transmission reduces dramatically from 75% to 0% between 20 and 25°C, showing that the cloud point has been reached within this temperature range. This is consistent with the manufacturer's quoted value of 24°C. (If a more precise value of the cloud point is required, measurements can be made with smaller temperature intervals, e.g. 1 or 2°C.) In contrast, J633 shows very little change in turbidity since it does not have a cloud point. J647 is therefore a suitable antifoam for use in the present invention, whereas J633 is not.

Example 2: Removal of antifoam from a model solution

An experiment was performed to demonstrate that antifoams can be removed from model solutions by raising the temperature of the solution above the cloud point, and removing the precipitate by filtration. A 0.3% (w/v) solution of Struktol J647 was prepared by taking an aliquot of 3.00g Struktol J647 and diluting to 1L with MilliO water. Samples of this solution were heated to above the cloud point by placing them in a water bath set at the required temperature for 1 hour. Samples were then gently mixed by swirling and filtered immediately.

Two different experiments were performed. Firstly, the effect of filter pore size was investigated using filters with pore sizes of 0.45μm (Pall Life Sciences Acrodisc), 0.2μm, 1.20μm and 5.00μm (all Sartorius Minisart) with a 2ml syringe at a fixed solution temperature (50°C). Secondly, the temperature of the solution was varied from 30 to 70°C (i.e. from 6 to 46°C above the cloud point) whilst using a fixed pore size (0.2 μm).

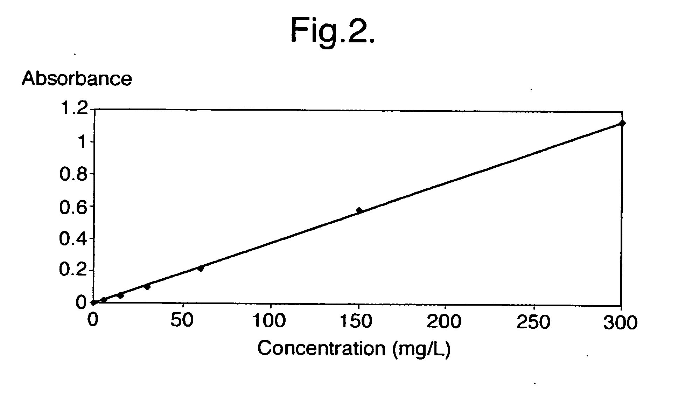

The concentrations of the antifoam in the filtrates were determined by using the Lange LCK 433 Water Testing Kit for non-ionic surfactants. This uses the principle that non-ionic surfactants (such as J647) form complexes with the indicator TBPE (tetrabromophenolphthalein ethyl ester), which can be extracted in dichloromethane and photometrically measured to determine the concentration. First, a calibration curve was constructed. A 0.3% (w/v) solution of Struktol J647 was prepared by taking an aliquot of 3.00 g Struktol J647 and diluting to 1 L with MilliQ water at 15°C. Aliquots were taken from this and diluted with MilliQ water to give concentrations of: 6, 15, 30, 60, 150 and 300 mg/L. MilliQ water was used as a blank sample. 0.2ml samples of each concentration were added to the kit test tubes containing TBPE and dichloromethane. The tubes were gently mixed for 2 minutes and allowed to stand for 30 minutes. They were then measured in a Lange DR2800 spectrophotometer in at 605nm in accordance with the Testing Kit instructions. Figure 2 shows the resulting calibration graph.

The filtrates were then diluted 1/10 with MilliQ water. 0.2 ml samples were measured in the spectrophometer as before, and the concentration of the antifoam in each filtrate was read off from the calibration graph. The amount (%) of antifoam remaining in the filtrate was calculated as

$$\text{(measured concentration in filtrate) / (known starting concentration)} \times 100\%.$$

Antifoam concentrations down to 0.2mg/L ($2\times10^{-5}$ % w/v) can be measured by a similar technique, using the Lange LCK 333 Water Testing Kit, and constructing a calibration curve in the appropriate concentration range. In this case a 2ml aliquot of the sample to be measured is added to the test kit, rather than 0.2ml.

The results are given in Table 2. The difference in the amount of antifoam remaining between the two measurements using the 0.2*m filter at 50°C (i.e. 6%) indicates the error bar associated with this method.

Table 2

| Filter pore size (μm) | Solution temperature (°C) | Temperature above cloud point (°C) | Remaining antifoam (%) |
|---|---|---|---|
| 0.2 | 50 | 26 | 6 |
| 0.45 | 50 | 26 | 17 |
| 1.2 | 50 | 26 | 28 |
| 5.0 | 50 | 26 | 79 |
| | | | |
| 0.2 | 30 | 6 | 26 |
| 0.2 | 50 | 26 | 12 |
| 0.2 | 70 | 46 | 9 |

The data show that the smaller the filter pore size, the greater is the amount of antifoam removed, i.e. the amount remaining in the solution is decreased, as expected. For J647, a pore size of 5.0μm is not small enough to remove most of the antifoam, whereas a pore size of 0.2 μm results in the removal of about 90% of the antifoam. The data also show that for a given pore size, increasing the solution temperature results in more effective antifoam removal.

Example 3. Removal of antifoam from a model fermentation medium

To demonstrate the removal of an antifoam from a typical fermentation medium, a model fermentation medium was prepared. First, two solutions having the compositions shown in Table 3 were prepared. In a typical fed-batch fermentation Batch 1 would be the starting medium and Batch 2 would be fed gradually through the feed-period.

Table 3

| Ingredient | Batch 1 (g/L) | Batch 2 (g/L) |
|---|---|---|
| Glucose | 22 | 440 |
| Galactose | 0 | 10 |
| Yeast extract | 10 | 25 |
| Potassium dihydrogen orthophosphate | 2.1 | 12 |
| Magnesium Sulphate | 0.6 | 2.5 |
| Antifoam - Struktol J647 | 0.4 | 0.8 |
| milliQ water | to1L | to1L |

Each batch (1 L volume) was autoclaved for 20 minutes at 121°C. The batches were then mixed (50:50) to give a model fermentation medium with an antifoam concentration of 0.6g/L. The medium was not inoculated or subjected to fermentation, but was tested in its raw form. Samples were heated and filtered to remove the antifoam, and the remaining amount of antifoam was measured, all as described in example 2. The proportion of antifoam remaining in each case is given in Table 4.

Table 4

| Filter pore size (μμm) | Solution temperature (°C) | Temperature above cloud point (°C) | Remaining antifoam (%) |
|---|---|---|---|
| 0.2 | 30 | 6 | 16 |
| 0.2 | 50 | 26 | 10 |
| 0.2 | 70 | 46 | 6 |
| 0.45 | 50 | 26 | 8 |
| 1.2 | 50 | 26 | 10 |

The experiment was repeated, but additional antifoam was added to the model fermentation medium so that the starting concentration was 3g/L. The results are shown in Table 5.

Table 5

| Filter pore size (μm) | Solution temperature (°C) | Temperature above cloud point (°C) | Remaining antifoam (%) |
|---|---|---|---|
| 0.2 | 50 | 26 | 8 |
| 0.45 | 50 | 26 | 8 |
| 1.2 | 50 | 26 | 15 |

This demonstrates that by selecting an antifoam which has a cloud point, the antifoam can be substantially removed from the model fermentation medium in a simple and convenient manner.

Example 4: Removal of antifoam from a fermentation liquor containing a foaming agent

A fed-batch fermentation of a genetically modified strain of *Saccharomyces cerevisiae* was performed. The strain had been modified by incorporating the gene encoding the hydrophobin HFBII from the fungus *Trichoderma reesei* (a foaming agent) in such a way that extracellular expression of the hydrophobin was achieved during fermentation. Fermentation was carried out essentially as described by van de Laar T et al., in Biotechnol Bioeng. 96(3):483-94 (1997), using glucose as a carbon source and scaling the process to a total volume of 150L in a 300L fermentation vessel. The antifoam Struktol J647 was used to control foaming during the fermentation (instead of Struktol J673 used by van de Laar T *et al).*

At the end of the fermentation, the fermentation liquor was microfiltered at 15°C (i.e. below the cloud point of the antifoam J647) to remove the yeast cells. Microfiltration was performed on pilot scale plant with Kerasep ceramic membranes having a pore size of $0.1\mu m$, using two volumes of diafiltration with deionised water. The liquor was then ultrafiltered, again at 15°C, to partially purify the HFBII. Ultrafiltration was by 1 kD Synder spiral wound polymeric membranes at a transmembrane pressure of 0.9 bar and four volumes of diafiltration.

The concentration of the antifoam in the fermentation liquor after the ultrafiltration step was measured (as described in example 2) to be 0.196g/L. The concentration of HFBII was measured to be 0.320g/L by high performance liquid chromatography (HPLC), as follows. The sample was diluted with 60% aqueous ethanol to give an approximate concentration of 200 $\mu$g/ml prior to analysis. HPLC separation was performed on a Vydac Protein C4 column (250 x 4.6 mm) at 30°C. Hydrophobin was measured by UV detection at 214nm and the concentration was calculated by comparison with samples of known HFBII concentration obtained from VTT Biotechnology (Espoo, Finland).

The cell-free liquor was then heated to 50°C, held at that temperature for 30 minutes and then filtered ($0.2\mu m$ pore size) to remove the antifoam, as described in example 2. The remaining amounts of antifoam and HFBII in the filtrate were measured as before and are given in Table 6 (column headed "Stage 1 "). The filtrate from this first stage was then re-heated to 50°C, held at this temperature for a further 30 minutes, and filtered as before. The HFBII and antifoam concentrations in the resulting filtrate were measured and are also given in Table 6 ("Stage 2").

Table 6

|  | Stage 1 | Stage 2 |
|---|---|---|
| Amount of HFBII in filtrate (g/L) | 0.32 | 0.30 |
| % of HFBII remaining | 100 | 93.75 |
| Amount of antifoam in filtrate (g/L) | 0.05 | 0.028 |
| % of antifoam remaining | 25.5 | 14.3 |
| Mass ratio of antifoam:HFBII | 0.156 | 0.093 |

This demonstrates that by selecting an antifoam which has a cloud point, the antifoam can be substantially removed from a fermentation liquor containing host cells and a foaming agent in a simple and convenient manner.

**Claims**

1.  A method for producing a foaming agent comprising:

    i) cultivating a host cell in a fermentation medium wherein:

       the host cell extra-cellularly secretes a foaming agent; and
       the fermentation medium contains an antifoam which has a cloud point;

    ii) removing the antifoam while the temperature of the fermentation medium is above the cloud point.

2.  A method according to claim 1 wherein in step i) the fermentation medium is aerated by sparging air or oxygen-enriched air into it.

3.  A method according to claim 1 or claim 2 wherein in step i) the temperature of the fermentation medium is above the cloud point of the antifoam.

**4.** A method according to any of claims 1 to 3 wherein in step ii) the antifoam is removed by filtration, centrifugation or adsorption.

**5.** A method according to claim 4 wherein the antifoams is removed by membrane filtration.

**6.** A method according to any of claim 1 to 5 wherein at least 75% of the antifoam is removed in step ii).

**7.** A method according to any of claims 1 to 6 wherein the temperature of the fermentation medium is at least 10°C above the cloud point in step ii).

**8.** A method according to any of claims 1 to 7 wherein the host cells are removed from the fermentation medium in step ii).

**9.** A method according to any of claims 1 to 8 wherein the foaming agent is purified and/or concentrated from the fermentation medium after step ii).

**10.** A method according to any of claims 1 to 9 wherein the antifoam is food grade.

**11.** A method according to any of claims 1 to 10 wherein the antifoam comprises at least one non-ionic surfactant/polymer.

**12.** A method according to claim 11 wherein the antifoam is a polyether, a poly(alkylene glycol), an ethylene/propylene oxide block co-polymer, a polyalcohol based on EO/PO block co-polymer, a polypropylene glycol based polyether dispersion, or an alkoxylated fatty acid ester.

**13.** A method according to any of claims 1 to 12. wherein the foaming agent is food grade

**14.** A method according to any of claims 1 to 13 wherein the foaming agent is a hydrophobin.

**15.** A method according to any of claims 1 to 14 wherein the host cell is a genetically-modified fungus.

**16.** A method according to any of claims 1 to 15 wherein the weight ratio of antifoam to foaming agent after step ii) is less than 0.2.

**Patentansprüche**

**1.** Verfahren zum Herstellen eines Schaumbildners, das Folgendes aufweist:

i) Züchten einer Wirtszelle in einem Fermentationsmedium, wobei die Wirtszelle einen Schaumbildner extrazellulär ausscheidet und das Fermentationsmedium ein Schaumverhütungsmittel enthält, das einen Trübungspunkt aufweist;
ii) Entfernen des Schaumverhütungsmittels, wobei die Temperatur des Fermentationsmedium oberhalb des Trübungspunktes gehalten wird.

**2.** Verfahren nach Anspruch **1,** wobei im Schritt i) das Fermentationsmedium belüftet wird, indem Luft oder mit Sauerstoff angereicherte Luft eingeblasen wird.

**3.** Verfahren nach Anspruch 1 oder Anspruch 2, wobei im Schritt i) die Temperatur des Fermentationsmediums oberhalb des Trübungspunkts des Schaumverhütungsmittels liegt.

**4.** Verfahren nach einem der Ansprüche 1 bis 3, wobei im Schritt ii) das Schaumverhütungsmittel durch Filtration, Zentrifugieren oder Adsorption entfernt wird.

**5.** Verfahren nach Anspruch 4, wobei das Schaumverhütungsmittel durch Membranfiltration entfernt wird.

**6.** Verfahren nach einem der Ansprüche 1 bis 5,
wobei im Schritt ii) mindestens 75 % des Schaumverhütungsmittels entfernt werden.

**7.** Verfahren nach einem der Ansprüche 1 bis 6,
wobei die Temperatur des Fermentationsmediums im Schritt ii) mindestens 10 °C über dem Trübungspunkt liegt.

**8.** Verfahren nach einem der Ansprüche 1 bis 7,
wobei die Wirtszellen im Schritt ii) aus dem Fermentationsmedium entfernt werden.

**9.** Verfahren nach einem der Ansprüche 1 bis 8,
wobei der Schaumbildner nach dem Schritt ii) aus dem Fermentationsmedium gereinigt und/oder konzentriert wird.

**10.** Verfahren nach einem der Ansprüche 1 bis 9,
wobei das Schaumverhütungsmittel Lebensmittelqualität hat.

**11.** Verfahren nach einem der Ansprüche 1 bis 10,
wobei das Schaumverhütungsmittel zumindest ein nichtionisches Tensid/Polymer aufweist.

**12.** Verfahren nach Anspruch 11,
wobei das Schaumverhütungsmittel ein Polyether, ein Poly(alkylenglycol), ein Ethylen/Propylenoxid-Blockcopolymer, ein auf einem EO/PO-Blockcopolymer basierender Polyalkohol, ein auf einer Polyetherdispersion basierendes Polypropylenglycol oder ein alkoxylierter Fettsäureester ist.

**13.** Verfahren nach einem der Ansprüche 1 bis 12,
wobei der Schaumbildner Lebensmittelqualität hat.

**14.** Verfahren nach einem der Ansprüche 1 bis 13,
wobei der Schaumbildner Hydrophobin ist.

**15.** Verfahren nach einem der Ansprüche 1 bis 14,
wobei die Wirtszelle ein genetische modifizierter Pilz ist.

**16.** Verfahren nach einem der Ansprüche 1 bis 15,
wobei das Gewichtsverhältnis zwischen dem Schaumverhütungsmittel und dem Schaumbildner nach dem Schritt ii) weniger als 0,2 beträgt.

**Revendications**

**1.** Procédé de production d'un agent moussant comprenant :

i) la culture d'une cellule hôte dans un milieu de fermentation dans lequel :

la cellule hôte secrète de manière extracellulaire un agent moussant ; et
le milieu de fermentation contient un antimousse qui possède un point de trouble ;

ii) le retrait de l'antimousse alors que la température du milieu de fermentation est au-dessus du point de trouble.

**2.** Procédé selon la revendication 1, dans lequel dans l'étape i), le milieu de fermentation est aéré par barbotage d'air ou d'un air enrichi en oxygène dans celui-ci.

**3.** Procédé selon la revendication 1 ou la revendication 2, dans lequel dans l'étape i), la température du milieu de fermentation est au-dessus du point de trouble de l'antimousse.

**4.** Procédé selon l'une quelconque des revendications 1 à 3, dans lequel dans l'étape ii), l'antimousse est retiré par filtration, centrifugation ou adsorption.

**5.** Procédé selon la revendication 4, dans lequel l'antimousse est retiré par filtration sur membrane.

**6.** Procédé selon l'une quelconque des revendications 1 à 5, dans lequel au moins 75 % de l'antimousse sont retirés dans l'étape ii).

**7.** Procédé selon l'une quelconque des revendications 1 à 6, dans lequel la température du milieu de fermentation est au moins 10 °C au-dessus du point de trouble dans l'étape ii).

**8.** Procédé selon l'une quelconque des revendications 1 à 7, dans lequel les cellules hôtes sont retirées du milieu de fermentation dans l'étape ii).

**9.** Procédé selon l'une quelconque des revendications 1 à 8, dans lequel l'agent moussant est purifié et/ou concentré à partir du milieu de fermentation après l'étape ii).

**10.** Procédé selon l'une quelconque des revendications 1 à 9, dans lequel l'antimousse est de qualité alimentaire.

**11.** Procédé selon l'une quelconque des revendications 1 à 10, dans lequel l'antimousse comprend au moins un polymère/tensioactif non ionique.

**12.** Procédé selon la revendication 11, dans lequel l'antimousse est un polyéther, un poly(alkylène glycol), un copolymère séquencé d'oxyde d'éthylène/oxyde de propylène, un polyol basé sur un copolymère séquencé d'EO/PO, une dispersion de polyéther à base de polypropylène glycol, ou un ester d'acide gras alcoxylé.

**13.** Procédé selon l'une quelconque des revendications 1 à 12, dans lequel l'agent moussant est de qualité alimentaire.

**14.** Procédé selon l'une quelconque des revendications 1 à 13, dans lequel l'agent moussant est une hydrophobine.

**15.** Procédé selon l'une quelconque des revendications 1 à 14, dans lequel la cellule hôte est un champignon génétiquement modifié.

**16.** Procédé selon l'une quelconque des revendications 1 à 15, dans lequel le rapport en poids de l'antimousse à l'agent moussant après l'étape ii) est inférieur à 0,2.

# Fig.1.

# Fig.2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1623631 A
- WO 0174864 A
- WO 9641882 A
- WO 01014521 A
- WO 0157076 A

### Non-patent literature cited in the description

- **BAILEY et al.** *Appl. Microbiol. Biotechnol.,* 2002, vol. 58, 721-727
- **DAVIS et al.** *Enzyme and Microbial Technology,* 2001, vol. 28, 346-354
- **SAMBROOK et al.** Molecular Cloning: A Laboratory Manual. Cold Spring Harbor Laboratory Press, 2001
- **AUSUBEL et al.** Short Protocols in Molecular Biology. John Wiley & Sons, Inc, 1999
- **MACCABE ; VAN ALFEN.** *App. Environ. Microbiol,* 1999, vol. 65, 5431-5435
- **DESAI ; BANAT.** *Microbiology and Molecular Biology Reviews,* March 1997, 47-64
- **WESSELS.** *Adv. Microb. Physio.,* 1997, vol. 38, 1-45
- **WOSTEN.** *Annu Rev. Microbiol.,* 2001, vol. 55, 625-646
- **DE VOCHT et al.** *Biophys. J.,* 1998, vol. 74, 2059-68
- **WOSTEN et al.** *Embo. J.,* 1994, vol. 13, 5848-54
- **TALBOT.** *Curr. Biol,* 2003, vol. 13, R696-R698
- Surfactant Aggregation and Adsorption at Interfaces. **J. EASTOE.** Colloid Science: Principles, Methods and Applications. Blackwell Publishing, 2005, 63
- Separation Processes in the Food and Biotechnology Industries. 7
- **YAMAGIWA et al.** *J. Chem. Eng. Japan,* 1993, vol. 26, 13-18
- **COLLEN et al.** *Biochim Biophys Acta,* 2002, vol. 1569, 139-50
- **CALONJE et al.** *Can. J. Microbiol.,* 2002, vol. 48, 1030-4
- **ASKOLIN et al.** *Appl Microbiol Biotechnol.,* 2001, vol. 57, 124-30
- **DE VRIES et al.** *Eur J Biochem.,* 1999, vol. 262, 377-85
- **VAN DE LAAR T et al.** *Biotechnol Bioeng.,* 1997, vol. 96 (3), 483-94